# EUROPEAN PATENT APPLICATION

(11) **EP 2 859 885 A2**
(43) Veröffentlichungstag der Anmeldung: **15.04.2015**
(21) Anmeldenummer: 13800655.6
(22) Anmeldetag: 06.06.2013
(51) Int. Cl.: A61K 9/10, A61K 31/7048, A61P 31/00

(54) **ANTIBAKTERIELLE PHARMAZEUTISCHE ZUSAMMENSETZUNG**

(30) Priorität: 08.06.2012 RU 2012123616
(71) Anmelder: Obshchestvo S Ogranichennoy Otvetstvennostyu "Vik - Zdorovye Zhivotnykh", Moscow 125299 (RU)
(72) Erfinder: VIOLIN, Boris Viktorovich, Moscow 123022 (RU); SEMENOV, Sergey Vyacheslavovich, Saratov 410019 (RU); PRISTENSKIY, Dmitriy Vladimirovich, Saratovskaya obl. Saratovskiy r-n. pos. Ust-Kurdyum 410540 (RU); ANISKOV, Aleksandr Andreevich, Saratov 410031 (RU)
(74) Vertreter: Spengler, Robert
(86) Internationale Anmeldenummer: PCT/RU2013/000467
(87) Internationale Veröffentlichungsnummer: WO 2013/184037

(57) **Zusammenfassung**

Die Erfindung betrifft Bereiche der Medizin und der Veterinärmedizin und kann zur Prophylaxe und Therapie bakterieller Infektionen eingesetzt werden. Antibakterielle pharmazeutische Zusammensetzung zum Injizieren umfasst Azithromycin, Lösungsmittel und/oder Co-Lösungsmittel, ein Konservierungsmittel und einen Oxydationshemmer. Als Oxydationshemmer werden Askorbinsäure oder Calciumascorbat oder Natriumascorbat oder Palmitoyl-ascorbat oder 4-Methyl-2 ,6-ditretbutilfenol oder Tretbutilgidrohinon, 2,4,5 Trihydroxybutyrophenon oder Natriumdisulfit oder Alpha-Tocopherol oder Thioglyzerin oder ihre Mischungen verwendet, während als Konservierungsmittel Benzylalkohol, Paraben, Chloreton oder ihre Mischungen und als Co-Lösungsmittel organisches Lösemittel, und zwar Dimethylazetamid oder N-Methyl-2-pyrrolidon oder 2-Pyrrolidon oder ihre Mischungen oder Mischungen organischer Co-Lösungsmittel mit Wasser auftreten, wobei folgende Massen- bzw.- Mengenverhältnisse vorliegen, Angaben in %:
Azithromycin 5-50,
Oxydationshemmer 0,1-0,2,
Konservierungsmittel 1-2,
Lösemittel - der Rest.

## Beschreibung

Die Erfindung betrifft Bereiche der Medizin und der Veterinärmedizin und kann zur Prophylaxe und Therapie bakterieller Infektionen eingesetzt werden.

Azithromycin (2R,3S,4R,5R,8R,10R,11R,12S,13S,14R)-13-[(2,6-Dihydroxy-3-C-methyl-3-O-methyl-a-L-ribohexopyranosil)oxy]- 2-Ethyl-3,4,10-trihydroxy-3,5,6,8,10,12,14-heptamethyl-11-[[3,4,6-trideoxy-3-(Dimethylamin)-b-D-Xylo-Hexapiranozil]Oxy]-1-ox-6-azatstklopentadekan -OH) ist ein halbsynthetisches Antibiotikum, ein Erythromyzinderivat. Azithromycin ist wirksam gegenüber grampositiven Mikroorganismen: Streptococcusspp. (Gruppen C, F und G, ausgenommen die gegen Erythromyzin resistenten), Streptococcuspneumoniae, Streptococcuspyogenes, Streptococcusagalactiae, Streptococcusviridans, Staphylococcusepidermidis, Staphylococcusaureus; gramnegative Bakterien: Haemophilusinfluenzae, Moraxellacatarrhalis, Bordetellapertussis, Bordetellaparapertussis, Legionellapneumophila, Haemophilusducreyi, Campylobacterjejuni, Neisseriagonorrhoeae und Gardnerellavaginalis; einigen Anaerobiern: Bacteroidesbivius, Clostridiumperfringens, Peptostreptococcusspp; sowie Chlamydiatrachomatis, Chlamydiapneumoniae, Mycoplasmapneumoniae, Mycobacteriumaviumcomplex, Ureaplasmaurealyticum, Treponemapallidum, Borreliaburgdorferi.

Bekannt ist eine flüssige pharmazeutische Zusammensetzung von Azithromycin zum Einsatz im Bereich Ophthalmologie, die bis zu 10% Wirkstoff enthält (US Nr. 6,277,829 B1 vom 21.08.2001 AntonioAsero, GraziaMazzone, process for preparation of aqueous formulation for ophthalmicuse). Bekannt ist auch eine flüssige pharmazeutische Zusammensetzung von Azithromycin für Lokaltherapie (US Nr. 7,064,104 B1 vom 20.06.2006 JacquesLuyckx, FredericPilotaz, Pharmaceutical composition based on macrolides for topical application in ophthalmology).

Bekannt ist eine schnell lösliche Form von Azithromycin zur peroralen Verabreichung (US Nr. 7,572,773 B2 vom 11.08.2009 AleksandarDanilovsky, KnezevieZdravka, single dose fast dissolving a zithromycin).

Es wurden Azithromycinsalze mit Malonsäure abgesondert. Diese Verbindungen können für das Ansetzen schnell löslicher Formen von Azithromycin zur peroralen Verabreichung verwendet werden. (US Nr.2009/0318375 A1 B2 vom 24.12.2009 Bo SungKwon, EunSookKim, Hee CheolKim, SangminYun, Myoung-sill Ko, Tae HunSong, HanKyongKim, KweeHyunSuh, GwansumLee, crystallineazithromycin L-malatemonohydrate and pharmaceutical composition containing same).

Bekannt sind verschiedene feste pharmazeutische Zusammensetzungen von von Azithromycin zur peroralen Verabreichung (US Nr. 2007/0185194 A1 vom 9.08.2007 KamalMehta, RajeevShankarMathur, SujataPaul, SanjeevKumarSethi, RajivMalik, stable oral compositions of azithromycin monohydrate; US Nr. 2007/0185194 A1 vom 9.08.2007 KamalMehta, RajeevShankarMathur, SujataPaul, SanjeevKumarSethi, RajivMalik, stable oral compositions of azithromycin monohydrate; US Nr. 2005/0106239 A1 vom 19.05.2005 RuthTenengauzer, JosephSchwarz, JuliaHrakovsky, TaniaLessen, LevKhondo, MathiMathivanan, ClaudeSinger, MichalPesachovich, stabilized azithromycin compositions; US Nr.2008/0096831 A1 vom 24.04.2008 MohsenSadatrezaei, PabloDavila, GaryBarbera, stabilized azithromycin composition).

Alle oben genannten Zusammensetzungen sind für äußerliche Anwendung bestimmt.

Bekannt ist eine feste Arzneiform, die lyophilisiertes Azithromycin enthält. (US Nr. 0116336 vom 1.07.2006 ByungHoWoo, K. KeithKnwok, KangYongYang, lyophilized azithromycin formulation). Es gibt eine lyophilisierte Form von Azithromycin unter dem Markennamen "Sumamed", hergestellt von Pliva.

Diese Arzneiform stellt ein bakterienfreies Pulver zur Zubereitung einer Injektionslösung unmittelbar vor Anwendung dar, da sich das Antibiotikum in dieser Lösung schnell zersetzt.

Der vorgeschlagenen Lösung liegt die flüssige Form von Azithromycin am nächsten, die Ethylalkohol, Co-Lösungsmittel: Polyethandiol, Propylenglykol; pH-Regler (sieh CN 1613453 "Azithromycin injection and its preparation").

Aber diese Arzneiform enthält Ethylalkohol und darf somit weder intramuskulär noch subkutan injiziert werden, da dies zu einer Gewebsnekrose führen kann. Sie wird ausschließlich intravenös aufgelöst in Wasserlösung injiziert. Somit handelt es sich nicht um eine gebrauchsfertige Injektionslösung, sondern um ein Konzentrat, das in die Lösung für intravenöse Infusionen zugegeben wird.

Die vorliegende Erfindungsaufgabe besteht in der Entwicklung eines Medikaments auf der Basis von Azithromycin in Form einer Lösung mit antimikrobieller Breitenwirkung. Das Medikament soll in Form einer gebrauchsfertigen Inektionslösung zum intravenösen sondern auch zum intramuskulären, subkutanen sowie zum intrauterinen und intrazisternalen Injizieren vorliegen.

Ein technisches Ergebnis besteht in hoher physikochemischer Stabilität (mindestens 18 Monate) der flüssigen Arzneiform für die Verwendung zur sicheren Therapie und Prophylaxe von durch krankheitserzeugende Mikroben verursachten Erkrankungen.

Das in der Anmeldung angegebene technische Ergebnis lässt sich durch experimentell festgestellte unbekannte Kombinationen aus Oxydationshemmern, organischen Lösemitteln und pH-Reglern und Makrolidantibiotikum - Azithromycin.

In der vorliegenden Zusammensetzung ist der Wirkstoff in der Mischung aus dem organischen Lösemittel und Wasser unter Zugabe von Stoffen zur Stabilisierung der Lösungsazidität aufgelöst. Die Verhältnisse von dem organischen Lösemittel, Wasser und den pH-Reglern (zur Regelung der Azidität) sind dermaßen ausgewählt, dass maximale physikochemische Stabilität der Lösung bei minimalem Abbau des aufgelösten Azithromycins erreicht werden kann. Bei der Auswahl des organischen Lösemittels und des pH-Reglers wurde auf die minimale Toxizität bei intramuskulären und subkutanen Injektionen geachtet.

Die Erfindung wird anhand einer Grafik erläutert ist und Daten zum Abbau von Azithromycinlösungen enthält, die nach den angeführten Rezepturen angesetzt worden sind.

Das entwickelte Medikament stellt eine stabile Lösung des Wirkstoffes Azithromycin. Als pH-Regler treten Zitronensäure, Apfelsäure, Benzoesäure oder Bernsteinsäure oder ihre Mischungen auf. Als Konservierungsmittel werden Benzylalkohol, Paraben, Monohloreton oder ihre Mischungen verwendet. Als Oxydationshemmer treten Askorbinsäure oder Calciumascorbat oder Natriumascorbat oder Palmitoyl-ascorbat oder 4-Methyl-2,6-ditretbutilfenol oder Tretbutilgidrohinon, 2,4,5 Trihydroxybutyrophenon oder Natriumdisulfit oder Tocopherolacetat oder Thioglyzerin oder ihre Mischungen auf.

Die Arzneiform kann einen Lösungsvermittler enthalten. Als Lösungsvermittler werden Cremophor EL oder Cremophor ELP (Polyoxyäthylen - Glycerol - Triritsinoleat) oder Cremophor RH40 (Polyoxyäthylen- Glycerol - Trihydroxystearat) oder Tween 80 (Polyoxyethylensorbitanmonooleat) oder Solutol HS15 (Polyäthylenglykol -660-12-Hydroxystearat) oder Poloxamer F68 oder Poloxamer F127 (Polyoxyäthylen - Polyoxypropylen Blockmischpolymer) oder ihre Mischungen verwendet. Darüber hinaus kann die Arzneiform ein Anästhesierungsmittel enthalten. Als Anästhesierungsmittel tritt Lidocainhydrochlorid bis zu 0,5%.

Verfahren zur Ermittlung der Stabilität von Azithromycinlösungen. Der Gehalt von Azithromycin wurde unter Verwendung von Hochdruckflüssigkeitschromatographie anhand des Verfahrens aus dem Arzneibuch RF XII, FS "Azithromycin" gemessen.

Zur Begründung der Auswahl des optimalen Lösemittels wurde die Löslichkeit von Azithromycin in unterschiedlichen organischen Lösemitteln ermittelt. (Tabelle 1).

| Tabelle 1 | |
|---|---|
| Löslichkeit von Azithromycin in organischen Lösemitteln | |
| Lösemittel | Maximalkonzentration von Azithromycin in % |
| Wasser | unter 0,5 |
| Dimethylsulfoxid | 26,4 |
| 2-Pyrrolidon | 28,9 |
| N-Methyl-2-pyrrolidon | 31,2 |
| N-Ethyl-2-pyrrolidon | 25,8 |
| N-Ethoxy-2-pyrrolidon | 23,3 |
| N-Octyl-2-pyrrolidon" | 21,5 |
| Dimethylazetamid | 32,1 |
| 3,3-Dimethyl-2-pyrrolidon | 26,4 |
| Propylenglykol | 13,4 |
| Transcutol | 10,2 |

Die optimalen Lösemittel sind N-Methyl-2-pyrrolidon und Dimethylazetamid.

### Zubereitungsart

Azithromycin wird im jeweiligen Lösemittel bzw. Lösungsmittelgemisch suspendiert, anschließend wird ein pH-Regler, ein Lösungsvermittler, ein Anästhesierungsmittel, ein Konservierungsmittel, ein Oxydationshemmer dazugegeben.

Nachfolgend sind Zusammensetzungen von Arzneiformen angeführt, die optimale Kombination von Komponenten aufweisen:

### Beispiel 1

### Zusammensetzung mit Anästhesierungsmittel.

| Bezeichnung der Komponenten | Menge, Masse in % |
|---|---|
| Azithromycin | 10,0 |
| Lidocain Hydrochlorid | 0,1 |
| N-Methyl-2-pyrrolidon | 50,0 |
| Zitronensäure | 3,2 |
| Benzylalkohol | 1,0 |
| Methyl-2,6-ditretbutilfenol | 0,1 |
| Wasser | bis 100 |

### Beispiel 2

Hier die Rezeptur mit Lösungsvermittler (Polyoxyäthylen -Glizerol- Triritsinoleat) und einer entsprechenden Menge andere Komponenten angeführt.

| Bezeichnung der Komponenten | Menge, Masse in % |
|---|---|
| Azithromycin | 10,0 |
| Dimethylazetamid | 30,0 |
| Zitronensäure | 3,2 |
| Polyoxyäthylen -Glizerol- Triritsinoleat | 15,0 |
| Benzylalkohol | 1,0 |
| Natriumdisulfit | 0,1 |
| Wasser | bis 100 |

### Beispiel 3

Rezeptur mit der maximalen Menge von Lösungsvermittler und einer entsprechenden Menge andere Komponenten.

| Bezeichnung der Komponenten | Menge, Masse in % |
|---|---|
| Azithromycin | 10,0 |
| Dimethylazetamid | 35,0 |
| Zitronensäure | 3,0 |
| Polyoxyäthylen -Glizerol- Triritsinoleat | 30,0 |
| Benzylalkohol | 1,0 |
| Methyl-2,6-ditretbutilfenol | 0,1 |
| Wasser | bis 100 |

### Bespiel Nº 4

Rezeptur mit der maximalen Menge des organischen Lösemittels und einer entsprechenden Menge andere Komponenten.

| Bezeichnung der Komponenten | Menge, Masse in % |
|---|---|
| Azithromycin | 20,0 |
| Dimethylazetamid | 74,0 |
| Zitronensäure | 1,6 |
| Benzylalkohol | 1,0 |
| Methyl-2,6-ditretbutilfenol | 0,1 |
| Wasser | bis 100 |

Eine Erhöhung des Anteils des organischen Lösemittels auf über 80% hat eine Erhöhung toxischer Wirkung der Arzneiform zur Folge.

### Beispiel 5

Rezeptur mit der maximalen Menge des organischen Lösemittels und einer entsprechenden Menge andere Komponenten ohne pH-Regler.

| Bezeichnung der Komponenten | Menge, Masse in % |
|---|---|
| Azithromycin | 20,0 |
| Dimethylazetamid | 74,0 |
| Benzylalkohol | 1,0 |
| Methyl-2,6-ditretbutilfenol | 0,1 |
| Wasser | bis 100 |

Die Entfernung des Reglers aus der Zusammensetzung der Arzneiform wirkt sich negativ auf die Stabilität der Lösung aus (die wird schnell dunkel, die Konzentration des Wirkstoffes sinkt), darüber hinaus hat die Erhöhung des Anteils der organischen Lösung auf über 70% eine Verstärkung toxischer Wirkung zur Folge.

### Beispiel 6

Hier ist die Rezeptur ohne Zugabe des organischen Co-Lösemittels angeführt, die eine entsprechende Menge anderer Komponenten enthält und bei der das Poloxamer F127 als Lösungsvermittler auftritt.

| Bezeichnung der Komponenten | Menge, Masse in % |
|---|---|
| Azithromycin | 5,0 |
| Zitronensäure | 3,2 |
| Poloxamer F127 | 5,0 |
| Benzylalkohol | 1,0 |
| Natriumdisulfit | 0,1 |
| Wasser | bis 100 |

Die Reduzierung des Anteils des Lösungsvermittlers auf unter 5,0% hat eine sinkende Stabilität der Arzneiform (ein Niederschlag lässt sich feststellen) zur Folge, darüber hinaus führt die Reduzierung des Wirkstoffanteils zur Verringerung therapeutischer Wirksamkeit der Arzneiform.

### Beispiel 7

Hier ist die Rezeptur ohne Zugabe des organischen Co-Lösemittels angeführt, bei der das Poloxamer F127 als Lösungsvermittler auftritt und die sich von der im Beispiel 6 dargestellten durch einen größeren Azithromycinanteil unterscheidet.

| Bezeichnung der Komponenten | Menge, Masse in % |
|---|---|
| Azithromycin | 10,0 |
| Zitronensäure | 3,2 |
| Poloxamer F127 | 8,0 |
| Benzylalkohol | 1,0 |
| Natriumdisulfit | 0,1 |
| Wasser | bis 100 |

Die Erhöhung des Azithromycinanteils auf über 10,0% führt bei Verwendung des Poloxamers F127 als Lösungsvermittler zur Viskositätserhöhung der Lösung bei der Temperatur unter +15°C.

### Beispiel 8

Rezeptur mit der maximalen Menge des H-Reglers einer entsprechenden Menge andere Komponenten.

| Bezeichnung der Komponenten | Menge, Masse in % |
|---|---|
| Azithromycin | 10,0 |
| Dimethylazetamid | 35,0 |
| Zitronensäure | 5,0 |
| Benzylalkohol | 1,0 |
| Askorbinsäure | 0,1 |
| Wasser | bis 100 |

Die Erhöhung des pH-Regleranteils auf über 5% hat eine Erhöhung der Lösungsazidität zur Folge, wobei sich die lokalreizende Wirkung der Arzneiform verstärkt und die Abbaugeschwindigkeit der Ausgangskomponente zunimmt.

### Beispiel 9

Rezeptur mit dem maximalem Wirkstoffanteil und einer entsprechenden Menge anderer Komponenten.

| Bezeichnung der Komponenten | Menge, Masse in % |
|---|---|
| Azithromycin | 50,0 |
| Dimethylazetamid | 43,9 |
| Zitronensäure | 5,0 |
| Benzylalkohol | 1,0 |
| Askorbinsäure | 0,1 |
| Wasser | bis 100 |

Bei der Azithromycinkonzentartion über 50% wird die Lösung nicht stabil genug, dabei sind die Lösungen mit der Wirkstoffkonzentartion im Bereich 30%-50% genug zäh, was sich negativ auf Nutzungseigenschaften auswirkt.

### Beispiel 10

Hier ist die Rezeptur angeführt, bei der als organisches Lösemittel auftritt.

| Bezeichnung der Komponenten | Menge, Masse in % |
|---|---|
| Azithromycin | 10,0 |
| Benzoesäure | 3,2 |
| Benzylalkohol | 1,0 |
| Askorbinsäure | 0,1 |
| Wasser | bis 100 |

Die Formen, bei denen als Hauptlösemittel auftritt, zeichnen sich durch eine geringere Stabilität aus (ein Niederschlag lässt sich feststellen).

### Beispiel 11

Rezeptur, bei als organisches Lösemittel auftritt.

| Bezeichnung der Komponenten | Menge, Masse in % |
|---|---|
| Azithromycin | 10,0 |
| Benzoesäure | 3,2 |
| Benzylalkohol | 1,0 |
| Askorbinsäure | 0,1 |
| Wasser | bis 100 |

Formen, bei denen Benzylalkohol (oder andere Alkohole) als Co-Lösemittel auftreten, zeichnen sich durch eine geringere Stabilität aus (Veränderung der Farbe der Lösung und Abbau des Wirkstoffes).

Die Untersuchungen der Stabilität wurden für Muster durchgeführt, die bei der Umgebungstemperatur (17-26°C) gelagert wurden. Die Ergebnisse sind in der Tabelle 7 dargestellt.

| Tabelle 2 | | | | | |
|---|---|---|---|---|---|
| Exposition, Anzahl der Tage | 0 | 120 | 240 | 480 | 540 |

| Beispiel Nr. | Azithromycinakonzentration % | | | | |
|---|---|---|---|---|---|
| 1 | 10,17 | 10,08 | 9,98 | 9,86 | 9,76 |
| 2 | 10,05 | 9,89 | 9,57 | 9,08 | 8.98 |
| 3 | 10,10 | 9,78 | 9,46 | 9,13 | 9.04 |
| 4 | 20,68 | 20,32 | 19,86 | 18,78 | 18.59 |
| 5 | 20,46 | 19,59 | 17,98 | 15,96 | 14.99 |
| 6 | 5,34 | 5,29 | 5,18 | 5,06 | 4.96 |
| 7 | 10,17 | 10,03 | 9,86 | 9,64 | 9.49 |
| 8 | 10,22 | 9,47 | 8,68 | 5,46 | 3.99 |
| 9 | 50,54 | 49,06 | 48,03 | 45,67 | 44.42 |

Aus den dargestellten Daten folgt, dass nach den oben angeführten Rezepturen zubereiteten Arzneiformen den Anforderungen an die physikochemische Stabilität entsprechen, wobei die Rezepturen 1-4 die optimalen sind. Dabei lassen sich die physikochemischen Eigenschaften der Arzneiform durch die Aufnahme eines Anästhesierungsmittels in die Zusammensetzung keineswegs beeinflussen.

Aus der Grafik ist ersichtlich, dass die Zugabe einer unzureichenden Menge des pH-Reglers eine sinkende Stabilität der Lösung zur Folge hat (die Konzentration des Wirkstoffes sinkt, Beispiel 5), wobei eine überflüssige Menge zum gleichen Effekt führt (Beispiel 8). Eine Nichtaufnahme des organischen Lösemittels in die Injektionslösung bei der gleichzeitigen Verwendung des entsprechenden Lösungsvermittlers (Polaxamer F 127) wirkt sich nur schwach auf die Stabilität der Lösung (Beispiele 6, 7). Die Aufnahme eines Lösungsvermittlers in die Lösung bei der gleichzeitigen Verwendung eines organischen Lösemittels beeinflusst keineswegs die Stabilität (Beispiele 1-4), jedoch kann die das Vorhandensein von Oberflächenaktivstoffen pharmakologische Eigenschaften der gewonnenen Arzneiformen verbessern. Die Erhöhung des Azithromycinanteils bis 50% (Beispiel 9) setzt nur die Verwendung des organischen Lösemittels mit einem pH-Regler voraus, dabei ist der Einsatz von Oberflächenaktivstoffen ausgeschlossen, da die Formen, die einen Lösungsvermittler enthalten, eine hohe Viskosität aufweisen.

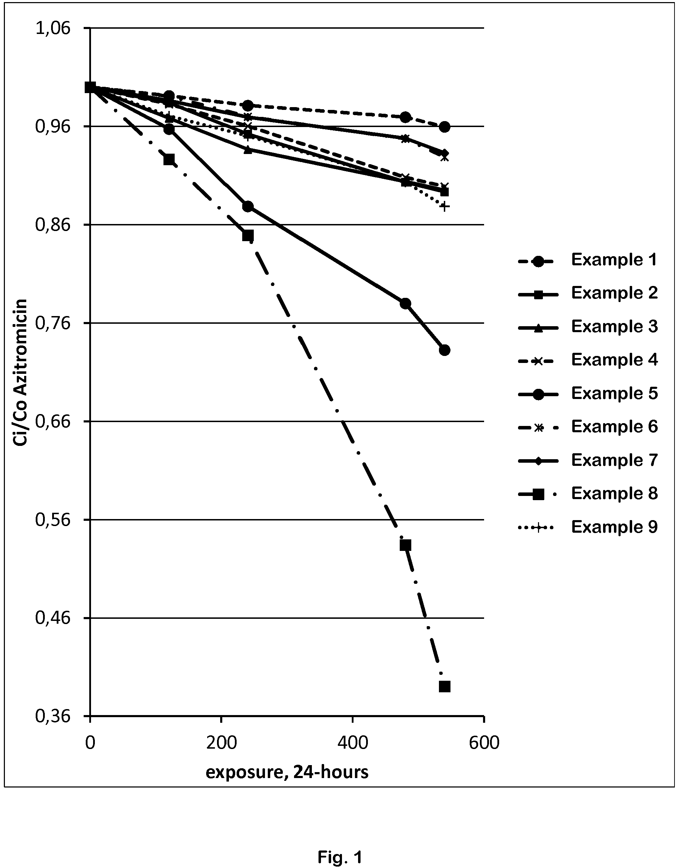

## Patentansprüche

1. Antibakterielle pharmazeutische Zusammensetzung, die Azithromycin, Lösungsmittel und/oder Co-Lösungsmittel, ein Konservierungsmittel umfasst, **dadurch gekennzeichnet, dass** diese einen Oxydationshemmer enthält, der durch Askorbinsäure oder Calciumascorbat oder Natriumascorbat oder Palmitoyl-ascorbat oder 4-Methyl-2 ,6-ditretbutilfenol oder Tretbutilgidrohinon, 2,4,5 Trihydroxybutyrophenon oder Natriumdisulfit oder Alpha-Tocopherol oder Thioglyzerin oder ihre Mischungen vertreten ist, während als Konservierungsmittel Benzylalkohol, Paraben, Chloreton oder ihre Mischungen, und als Co-Lösungsmittel organisches Lösemittel ausgewählt sind, Angaben in %:
| | |
|---|---|
| Azithromycin | 5-50 |
| Oxydationshemmer | 0,1-0,2 |
| Konservierungsmittel | 1-2 |
| Lösemittel | Der Rest |

2. Zusammensetzung nach Anspruch 1, **dadurch gekennzeichnet, dass** das organische Lösungsmittel als Lösungsmittel oder Co-Lösungsmittel verwendet wird: Dimethylacetamid oder Dimethylsulfoxid oder N-Methyl-2-pyrrolidon oder N-Ethyl-2-pyrrolidon oder N-Ethoxy-2-pyrrolidon oder N-Octyl-2 -pyrrolidon oder 3,3-Dimethyl-2-pyrrolidon, Propylenglykol oder Transcutol (monoethylether Diethylenglykol) oder Benzylalkohol, oder Kombinationen davon; oder Wasser oder eine Kombination von organischen Lösungsmitteln mit Wasser.

3. Zusammensetzung nach Anspruch 1, **dadurch gekennzeichnet, dass** diese zusätzlich einen pH-Einsteller in einer Menge bis zu 5% enthält.

4. Zusammensetzung nach Anspruch 1, **dadurch gekennzeichnet, dass** diese zusätzlich einen solublizer: Polyethylenglykol -660-12-Hydroxy-Stearat oder Poloxamer F 68 oder Poloxamer F 127 oder Polyoxyethylen-glycerin-triricinoleate oder Polyoxyethylen-glycerintrihydroxystearate oder Polyoxyethylensorbitanmonooleat oder eine Kombination davon in einer Menge von 1-30% enthält.

5. Zusammensetzung nach Anspruch 1, **dadurch gekennzeichnet, dass** diese zusätzlich ein Anästhetikum bis zu 2,0% enthält.

6. Zusammensetzung nach Anspruch 5, **dadurch gekennzeichnet, dass** Novocain oder sevicaine oder Bupivacain oder Lidocain oder Ropivacain, oder ein Gemisch davon als Anästhetikum verwendet wird.
